# EUROPEAN PATENT SPECIFICATION

(11) **EP 1 765 529 B1**
(45) Date of publication and mention of the grant of the patent: **20.07.2011**
(21) Application number: 05747248.2
(22) Date of filing: 27.05.2005
(51) Int. Cl.: B09C 1/10, E21B 43/22, C01B 3/24, C12P 3/00

(54) **PROCESS FOR STIMULATING PRODUCTION OF HYDROGEN FROM PETROLEUM IN SUBTERRANEAN FORMATIONS**
VERFAHREN ZUR STIMULIERUNG DER PRODUKTION VON WASSERSTOFF AUS ERDÖL IN UNTERIRDISCHEN FORMATIONEN
PROCEDE PERMETTANT DE STIMULER LA PRODUCTION D'HYDROGENE A PARTIR DU PETROLE DANS DES FORMATIONS SOUTERRAINES

(30) Priority: 28.05.2004 GB 0412059
(43) Date of publication of application: 28.03.2007
(73) Proprietor: University of Newcastle upon Tyne, Newcastle upon Tyne NE1 7RU (GB)
(72) Inventor: LARTER, Stephen Richard, Calgary, Alberta T3A 3T6 (CA); HEAD, Ian McCutcheon, Northumberland NE42 6AS (GB); JONES, D. M. 8 Edlingham Close, Tyne and Wear NE3 1RH (GB); ERDMANN, Michael, N-5221 Nesttun (NO); WILHELMS, Arnd, N-5243 Fana (NO)
(74) Representative: Watson, Robert James
(86) International application number: PCT/GB2005/002109
(87) International publication number: WO 2005/115648

(56) References cited:
- WO-A-01/68904
- WO-A-02/06503
- US-A- 5 464 539
- US-A1- 2004 050 778

## Description

This invention relates to a process of converting petroleum and other fossil fuels to hydrogen in a subterranean formation at an economically significant rate using microbial action and recovering the hydrogen.

### Background

'Petroleum' means crude oil including heavy and residual oils in any reservoir, bitumen in tar sands, natural gas, gas condensate and any hydrocarbon containing fluid producible through boreholes or solid and fluid hydrocarbon containing materials recoverable from mining of tar sands or bitumen containing reservoirs of any type.

Hydrogen is an important fuel and chemical process substrate of great economic importance. While it may or may not be the fluid transportation fuel of the future, hydrogen is already a major component of the world chemical industry with over 9.2 billion CuM hydrogen shipped in the US in 2002 worth over $750 billion. Hydrogen as a fuel does not produce significant greenhouse gas emission and is thus environmentally favoured over carbon based fuels. Currently hydrogen is mainly used for chemicals processing with over 50% derived from steam reforming of natural gas. Hydrogen will be needed for processing of the heavy oils in the early 21^{st} century as the very heavy tar sand bitumens require up to 80% more hydrogen to process than light conventional oils. Irrespective of its use as a transportation fuel, the hydrogen economy is already growing at 12-17% per year. Generating hydrogen from natural gas is wasteful and expensive while alternate sources such as electrolysis, or from nuclear-chemical processes are expensive or have environmental problems of their own. Direct generation of hydrogen within petroleum reservoirs would produce an environmentally neutral fuel and chemical feedstock with reduced carbon emission and provide a basis for a third generation petroleum industry with truly green credentials.

When oil is present in porous and permeable subterranean rock formations such as sandstone, carbonate, chert, shale or fractured rocks of any type, it can generally be exploited by drilling into the oil-bearing formation and allowing existing pressure gradients to force the oil through the reservoir and up the borehole. This process is known as primary recovery.

If and when the pressure gradients are insufficient to produce oil at the desired rate, it is customary to carry out an improved recovery method to recover additional oil. This process is known as secondary recovery.

There are several secondary recovery techniques, including gas injection and water injection. Choice of a specific secondary recovery technique depends on the specifics of the petroleum accumulation. Water injection or water flooding is the most common secondary recovery technique. In water flooding, pressurized water is injected into the oil-bearing formation and oil and gas is produced from neighbouring petroleum production wells. First petroleum, and subsequently petroleum and water are recovered from the production well.

However, even after secondary recovery, a significant portion of petroleum remains in the formation. With oil this is usually in excess of 40% and in some cases over 75% of the original oil in place. The fraction of unrecoverable petroleum is typically highest for heavy oils, tar, and petroleum in complex reservoir formations. Much of this remaining oil is trapped due to capillary or sorptive forces and represents an irreducible oil saturation. Additional oil is trapped as bypassed oil within the rock formation missed by primary and secondary recovery techniques. This remaining, or residual oil, may be recovered by enhanced recovery techniques. One enhanced oil recovery technique uses microorganisms (either indigenous or introduced) to dislodge the trapped or adsorbed oil from the rock. The goal of this technique, which is known as microbially enhanced oil recovery (MEOR), is to increase oil recovery of the original subsurface petroleums. MEOR processes typically use microorganisms to: (1) alter the permeability of the subterranean formation by blocking porethroats to divert water flow to regions still saturated with oil, (2) produce biosurfactants which decrease surface and petroleum/water interfacial tensions and mediate changes in wettability, (3) produce polymers which facilitate increased mobility of petroleum, (4) produce low molecular weight acids which cause rock dissolution and increase permeability, and (6) generate gases (predominantly CO₂) that increase formation pressure and reduce oil viscosity when dissolved in the oil.

Numerous microorganisms have been proposed for achieving various microbial objectives in subterranean formations. Most MEOR techniques involve injection and establishment of an exogenous microbial population into the oil-bearing formation. The population is supplied with growth substrates and mineral nutrients as additives to the waterflood used for secondary oil recovery. The growth of exogenous microorganisms is often limited by the conditions that prevail in the formation. Physical constraints, such as the small and variable formation pore throat sizes together with the high temperature, salinity and pressure of fluids in the formation and the low concentration of oxygen in the formation waters severely limit the types and number of microorganisms that can be injected and thrive in the formation. Biological constraints, such as competition from indigenous reservoir microbes and the stress of changing environments (from surface to subsurface) also acts to limit the viability of exogenously supplied microorganisms. To overcome these problems, indigenous microorganisms, commonly anaerobic organisms, have been proposed for use in MEOR techniques.

Microorganisms are commonly present in petroleum reservoirs cooler than about 80°C (Bernhard and Connan, 1992; Magot et al, 2000; Orphan et al, 2000; Wilhelms et al, 2001). Biodegradation of petroleum, both crude oil and natural gas, in the subsurface is a common process (Connan, 1984; James, 1984; Horstad and Larter, 1997; Wenger et al, 2001; Head et al, 2003 and refs therein). With appropriate environmental conditions and sufficient time, indigenous bacteria and archaea can convert petroleum or other fossil fuels such as coals to methane over long geological time periods in the subsurface (Scott et al, 1994; Head et al, 2003; Roling et al, 2003 and refs therein). Methanogenesis, an exclusively anaerobic process, is commonly associated with biodegraded petroleum reservoirs and isotopically lighter carbon containing methane is frequently found admixed with thermogenic methane (Scott et al., 1994; Larter et al., 1999; Sweeney and Taylor, 1999; Pallasser, 2000; Masterson et al., 2001; Boreham et al., 2001; Dessort et al, 2003) and methanogens represent common indigenous members of petroleum reservoir microflora (Mueller and Nielsen, 1996; Nilsen and Torsvik, 1996; Nazina et al., 1995 a,b; Ng et al., 1989). The methanogens described are those that reduce carbon dioxide to methane with few reports of acetoclastic methanogens from petroleum reservoirs (Obraztsova, 1987). Radiotracer experiments indicate that carbon dioxide reduction to methane is more prevalent than acetoclastic methanogenesis (Mueller and Nielsen, 1996; Rozanova et al., 1995) and high pressures in petroleum reservoirs favour net volume reducing reactions such as methanogenesis from carbon dioxide reduction (Head et al., 2003). The conversion process is slow under most geological conditions and it has been shown that typically it takes many millions of years to naturally biodegrade oil in a reservoir (Larter et al, 2003). Recent developments in microbiology have also demonstrated the existence of microbial consortia which can directly convert hydrocarbons to methane under conditions likely to be found in petroleum reservoirs (Zengler et al, 1999; Anderson and Lovely, 2000). This degradation is usually anaerobic in nature and methane is often the natural end product of oil degradation (Larter et al, 1999; Head et al, 2003) with a significant proportion of the methane produced being associated with the reduction of carbon dioxide using secondary sources of hydrogen (Röling et al 2003; Head et al, 2003). The hydrogen is generated by bacteria as part of the overall biodegradation process, this hydrogen represents an intermediate reactant in the overall conversion of petroleum to methane. The inventors consider hydrogen to be a normal and essential intermediate in natural oil and gas biodegradation under anoxic conditions.

The first order kinetic rate constants of biodegradation of hydrocarbons and non-hydrocarbons in petroleum reservoirs under natural conditions has been shown to be around 10⁻⁶ to 10⁻⁷/year approximately (Larter et al, 2003; Head et al, 2003) approximately 10,000 to 100,000 times slower than anaerobic hydrocarbon degradation rates in shallow subsurface environments such as landfills or shallow aquifers. The inventors consider that the rate-limiting step for petroleum biodegradation involves the initial attack by microorganisms on hydrocarbons and other molecules and it is during this stage that along with other intermediary metabolites, hydrogen production occurs via the action of fermentative, syntrophic bacteria. To commercially recover significant quantities of oil as hydrogen in realistic timescales of months to years using microbial technologies, accelerated biodegradation of large fractions of an oil layer to rates 10,000 times or more greater than natural rates of petroleum biodegradation, and subsequent consumption of hydrogen by sulphate reducing or methanogenic microorganisms and other hydrogen oxidizers, must be prevented. Further, to maintain hydrogen production hydrogen must be removed from the site of hydrogen generation otherwise the reaction becomes thermodynamically unfavourable. To produce commercial quantities of hydrogen by microbial degradation of petroleum in reservoirs under anaerobic conditions technologies for acceleration of hydrogen generation rates are needed and the degree of enhancement required to achieve commercial rates of production must be defined. Technologies are also needed for removal of the hydrogen from the site of reaction.

### Summary of the Invention

The present inventors have identified key steps for the identification of reservoirs capable of stimulation to produce hydrogen, of techniques for stimulation of hydrogen production, for specific acts needed to prevent hydrogen destruction by common reservoir microorganisms and techniques for removal of hydrogen from the site of generation to maintain active hydrogen production in petroleum reservoirs.

Accordingly, the present invention provides a process for stimulating microbial hydrogen production in a petroleum-bearing subterranean formation in which a microbial consortium comprising one or more hydrogen-consuming organisms is present, comprising:
(a) analyzing one or more components of the formation to determine characteristics of the formation environment;
(b) detecting the presence of a microbial consortium, comprising at least one fermentative syntrophic microorganism, within the formation;
(c) assessing whether the formation microrganisms are currently active;
(d) determining whether the microbial consortium comprises one or more fermentative syntrophic microorganisms;
(e) characterization of one or more fermentative syntrophic microorganism of the consortium, and comparing the one or more characterized organisms with at least one known characterized known microorganism having one or more known physiological and ecological characteristics;
(f) characterizations of said one or more hydrogen consuming microorganisms, such as methanogens, sulphate reducers, or other hydrogen-oxidizing organisms of the consortium, and comparing the one or more characterized microorganims with at least one known characterized, known microorganism having one or more known physiological and ecological characteristics;
(g) using information obtained from steps (a) through (e) for determining an ecological environment that promotes in situ microbial degradation of petroleum and promotes microbial generation of hydrogen by at least one fermentative syntrophic microorganism of the consortium;
(h) using information obtained from steps (a) and (f) , for determining an ecological environment that inhibits in situ microbial degradation of hydrogen by said at least one hydrogen-oxidizing microorganism of the consortium;
(i) modifying the formation environment based on the determinations of step (g) and (h) to stimulate microbial conversion of petroleum to hydrogen and inhibit microbial conversion of hydrogen to other compounds; and
(j) removal of the hydrogen generated from the sites of generation, so as to maintain an energetically feasible microbial conversion of petroleum to hydrogen.

The removal of hydrogen may be accomplished by a variety of means, including but not limited to using purge or sparge gases generated or injected into the reservoir. For example, designing the production system such that active gas generation of carbon dioxide or methane occurs below the hydrogen generating zones to flush the hydrogen away from the hydrogen generation zone to a production well where the hydrogen is produced admixed with methane and other gases produced from the reservoir.

It is preferred that the process includes as part of step (b) the step of detecting the presence of anaerobic hydrocarbon-degrading bacteria.

This process includes the steps of identifying whether oil layers are capable of active degradation with indigenous organisms or introduced organisms, whether fermentative syntrophic organisms that produce hydrogen are present and modifying the formation environment to increase their activity and whether methanogenic, sulphate reducing or other hydrogen-oxidizing microorganisms, that degrade hydrogen produced by the fermentative syntrophic microorganisms, are present, and modifying the formation environment to reduce their activity.

The process of this invention stimulates and sustains the activity of a mixture of different microorganisms in a petroleum-bearing, subterranean formation to convert petroleums to hydrogen, which can be produced. It also reduces the activity of one or more methanogenic, sulphate-reducing or other hydrogen-oxidizing organisms that are present, to avoid the degradation of the hydrogen produced. While not wishing to be bound by theory, it is believed that a mixture of microorganisms converts petroleums to methane and in the process generates hydrogen as an intermediate by multiple acts as follows:
(1) Microbial consortia degrade various petroleum compounds (e.g., saturated or aromatic hydrocarbons, asphaltenic, and nitrogen-sulphur-oxygen bearing organic compounds) by a single act or a series of acts into various compounds, which may include amines, alcohols, organicic acids, and gases including hydrogen and carbon dioxide.
(2) Methanogens convert various low molecular weight compounds, which may include amines, alcohols, organic acids, hydrogen and carbon dioxide into methane, CO₂, and water.

The present inventors have identified two classes of organisms in reservoirs which convert hydrogen into water and methane or hydrogen sulphide. These are the methanogens and the sulphate reducing bacteria. In addition to these, other hydrogen-oxidizing organisms may be present

The microorganisms naturally present in a subterranean formation will typically comprise mixed consortia of microorganisms, which will often depend on each other. For example, in the degradation of petroleum, syntrophic hydrogen-producing microorganisms obtain energy from petroleum degradation if their metabolic waste products (such as organic acids, acetate, and H₂) are continuously removed and kept at a relatively low concentration. Methanogenic microorganisms perform part of this waste-removal function by converting at least some of the waste products (for example, acetate, CO₂ and H₂) to methane.

This description of one embodiment of the invention will focus on converting petroleum to hydrogen in a conventional oil-bearing formation. However, the process of this invention can be applied to any petroleum-bearing formation in which environmental conditions can be modified to stimulate growth of at least one petroleum-degrading microorganism that is capable of converting the original petroleum or the degradation products to hydrogen. The process of this invention can be used to stimulate microbial activity in oil shale deposits, newly worked and abandoned coal seams, tar sands an other fossil fuel deposits to transform the organic compounds contained therein to hydrogen. Additionally the technique can be used to dispose of waste organic matter such as chemical wastes or sewage and in the process aid in the generation of fuel gases including hydrogen and methane from petroleum or directly from waste organic matter in reservoirs. As used in this -description, the term "fossil fuels" is used in a broad sense to include solid carbonaceous deposits such as kerogen, peat, lignite, and coal; liquid carbonaceous deposits such as oil; gaseous hydrocarbons; and highly viscous carbonaceous deposits such as bitumen and tar.

This process of the invention can also be applied to reclamation projects where petroleum-contaminated soils and aquifers can be treated to enhance microbial conversion of petroleums or petroleum or chemical products to recoverable hydrogen and other fuel gases.

In this description, indigenous microorganisms that transform petroleums to hydrogen are identified and then stimulated, whilst indigenous microorganisms that degrade hydrogen are identified and then suppressed.

The term "microorganisms" is intended to include bacteria and archaea organisms, their enzymes, and other products as well as relevant eukarya. It will be understood that bacteria and archaea are representative of microorganisms in general that can degrade petroleum and/or consume the resulting products under anoxic conditions.

### Brief Description of Figures

Figure 1 shows the processes involved in the transformation of oil into hydrogen in a petroleum reservoir, along with competing processes;
Figure 2 shows an ideal configuration for an oil and gas field to produce hydrogen and recover both residual oil and producible oil as hydrogen; and
Figure 3 shows a diagrammatic representation of an example of the invention.

### Analyzing the Fluid/rock Chemistry and Microbiology

In practicing the process of this invention, the first step is to analyze one or more samples of fluids (waters, oils and gases) and rocks in the petroleum-bearing formation in which microbial activity is to be stimulated. While one sample is sufficient to practice the invention, multiple samples may be obtained.

### Collecting Samples

The samples can be obtained by sampling procedures that are known to those skilled in the art. Normally, a fluid or gas sample is retrieved from the formation through perforations in a well casing or from an open-hole or production test. The fluids can be sampled either downhole with a wireline formation fluid tester or fluid sampler or at the surface wellhead from a subsurface test, such as drill stem tests, production tests, or normal production. Both formation water and petroleum (oil and gas) samples are useful for evaluation of the formation environment. Rock samples can be retrieved from drill cores, cuttings, produced sediments (including bailed samples) and/or outcrop sites or rock data can be secured by interpretation of well logs. Analyses from downhole chemical sensors may also be used.

### Environmental Analysis

An analysis of the formation's environment provides crucial information in determining suitable microbial growth stimulants or in situ environmental conditions for microbial activity. This analysis preferably includes determining the formation's temperature and pressure, which can be obtained in any suitable manner. While many reservoirs contain biodegraded oils, not all reservoirs contain currently active microbial populations. A key part of the process is the definition of reservoirs that contain relevant active organisms that can be stimulated, to recover economic levels of hydrogen through oil biodegradation.

To determine the environment in the reservoir, a geochemical analysis can be made of one or more fluids of the formation, such as formation water and petroleums, and/or one or more solids of the formation, which analyses are familiar to those skilled in the art. Preferably, the analysis is made of fluid and/or rock samples obtained from the formation. The fluid analysis can include measurement of the state values (for example, temperature and pressure) as well as a geochemical analysis of the formation water which can include assay for major anions and cations, pH, oxidation potential (Eh), chloride, sulphate, phosphate, nitrate, ammonium ion, sulphate, salinity, selenium, molybdenum, cobalt, copper, nickel, and other trace elements.

The geochemical analysis will preferably also identify products that are known to be produced by indigenous microbial activity. For example, presence of methane, CO₂, RNA, DNA, enzymes, and specific lipids and carboxylic acids can be indicative of microbial activity. Methane relatively depleted in the carbon 13 isotope is frequently found in oilfields where natural methanogenesis has occurred. In particular, anaerobic hydrocarbon degradation metabolites, such as alkyl and aryl substituted succinates or reduced naphthoic acids, are critical markers of systems in which the anaerobic degradation of hydrocarbons is taking place and a good indication of hydrogen production as an intermediate in the degradation process. The identification of such markers can be used as a first step in determining the presence of active anaerobic petroleum degrading microbial consortia.

A number of laboratory studies using aliphatic, aromatic, and polycyclic aromatic hydrocarbons as substrates for a variety of sulfate-reducing, denitrifying and methanogenic cultures have identified alkyl and aryl succinates, formed by the addition of fumarate either to a sub-terminal carbon of an alkane or to an alkyl substituent of an aromatic hydrocarbon, as the initial relatively stable metabolite in the degradation process (Widdel and Rabus, 2001; Wilkes, et al., 2002). Succinates have also been reported as metabolites from the biodegradation of both saturated and aromatic hydrocarbons in anoxic zones of petroleum-contaminated aquifers (Beller, *et al.*, 2002). A recent study of an anoxic zone in an aquifer contaminated with gasoline has also identified 2-naphthoic acid and reduced 2-naphthoic acids as evidence of anaerobic degradation (Annweiler, et al., 2002). Aitken, et al. (2002) have shown that actively degrading oilfields were found to contain 2-naphthoic acid and, more significantly, amounts of reduced 2-naphthoic acids, such as 5,6,7,8-tetrahydro-2-naphthoic acid which are exclusively indicators of anaerobic hydrocarbon degradation under the conditions appropriate for hydrogen generation. The presence of such compounds is indicative of anaerobic degradation conditions appropriate for hydrogen generation and subsequent methanogenesis.

Other compounds which are indicative of active hydrogen generation and methanogenesis under indigenous conditions are phospholipids characteristic of syntrophic bacteria and specific archaeols characteristic of hydrogenotrophic methanogenic archaea which the inventors have identified in fossil fuel deposits undergoing active biodegradation. Specific phospholipids and microbial DNA characteristic of syntrophic bacteria or methanogenic archaea can also be used to positively identify petroleum fields with active methanogenic processes that are capable of control and acceleration to commercial rates of hydrogen production.

An important feature of these analyses is that they should be focussed on the oil-water transition zones in reservoirs. Specific indicators of active petroleum degradation have been shown to be preferentially concentrated in samples near petroleum/water contacts and it is here that sampling and characterisation should be targeted.

Actively degrading petroleum reservoirs can also be identified by several geochemical proxies. Elevated carbon dioxide levels in produced gases, isotopically distinct methane enriched in the carbon 12 isotope, acidic metabolite markers as described above and crucially by the detection and measurement of compositional gradients in the oil columns. Gradients in oil columns such as variations in the saturated hydrocarbon contents versus depth in the oil layer have been detected in several oilfields by the authors and these have been used to assess the indigenous rates of hydrocarbon metabolism by reservoir microorganisms. The gradients are produced when organisms destroy hydrocarbons at the base of an oil column and the compositional profile of the oil column changes in response to this to produce a vertical and or lateral gradient in composition in such parameters including but not limited to saturated hydrocarbon content, n-alkane distribution or content or in the distribution of more resistant compounds such as isoprenoid alkanes or hopanes We claim here that the detection of such gradients can be used to identify fields where hydrogen generation can be accelerated as organisms are active where gradients are present. The rate of biological activity can be calculated from the gradient and thus indicate the extent to which acceleration of natural rates of degradation are required. This can be used to assess the extent of additive treatments necessary for enhancement of hydrogen generation to desired rates.

It is not only organic geochemical signatures that give indications of active processing of the oil naturally by microorganisms. High concentrations of metals such as Cobalt, Nickel or iron in the oils in the vicinity of the oil water contacts in the field's column are commonly found in reservoirs where active biodegradation has occurred and may be occurring.

Petroleum analyses will include quantitation of the major hydrocarbon types such as saturated hydrocarbons, aromatic hydrocarbons, resins and asphaltenes and detailed molecular characterisation of the specific hydrocarbon fraction such as n-alkanes, isoprenoid alkanes, alkylbenzenes, alkylnaphthalenes and so on. Chemical analyses of oil and gas will aid in identifying the abundances and compositions of the different carbon substrates for the microorganisms. While in principal many of the components of crude oils can be used for hydrogen production the most reactive oils and the fields most suitable for hydrogen generation will still contain abundant n-alkanes, isoprenoid alkanes and other more reactive components such as light alkanes and aromatic hydrocarbons. Analysis of petroleum extracted from produced fluids or cuttings or core samples taken through the oil column will allow chemical analyses to define the extent of any compositional gradients that exist in the oil column. Determination of the compositional gradients can be used to determine the current rates of biodegradation of the oil column and thus the extent to which biodegradation rates and methanogenesis rates in particular need to be accelerated.

The rock analysis may include mineralogical, chemical and facies descriptions as well as measurements of formation properties such as porosity, permeability, capillary pressure, and wettability.

### Microbial Analysis

### Collecting Indigenous Microorganisms

Correct sampling is a vital element of these analyses. Microbial populations in deep subsurface environments are typically very low and on the order of five to six orders of magnitude less abundant than in near surface sediments (ca. 10³ to 10⁴ cells per cubic centimetre in the deep subsurface). Thus to avoid misidentification of contaminant organisms as indigenous, it is essential that stringent contamination control measures are adopted. Treatment of all reagents and materials, except amplification primers, with UV and enzymatic treatment with DNase I are essential when nucleic acid based analyses are conducted. Samples for nucleic acid analysis should also be frozen immediately or fixed by addition of filtered 50% ethanol. Subsamples should be taken from the centre of whole cores under sterile conditions to avoid contamination from the exterior of the core contaminated during drilling. Samples for cultivation based studies should be stored either chilled or at close to *in situ* temperatures to reduce the growth of contaminating microorganisms during storage and transport. Ideally samples should be of core material to increase the likelihood of obtaining indigenous organisms free from contaminants however formation water and/or drill cutting samples may be analyzed for the presence of active microorganisms if conditions are maintained to inhibit exogenous contaminant organisms while promoting those adapted to in situ conditions. Microorganisms in water samples are preferably concentrated by filtration and/or centrifugation before the analysis is performed. The amount of the indigenous microbe population will typically be a small fraction of the sample's volume. In a typical oil-bearing formation, water may contain less -than 0.025 mg of microorganisms per liter. Alternatively, the indigenous microorganisms can be first cultured in the laboratory using techniques familiar to those skilled in the art and then concentrated and collected. Microorganism concentrations can be amplified to facilitate detection using conventional microbial detection techniques, which are familiar to those skilled in the art. Incubation of samples in microcosms that replicate as much as possible in situ conditions to identify factors that promote or inhibit particular metabolic processes is also a key approach to identifying candidate petroleum systems for successful microbial stimulation.

### Characterizing the Indigenous Microorganisms

Microorganism characterization as used in this description means identifying the key characteristics of a microorganism or consortium of microorganisms using one or more of the following methods: biochemical methods, physiological methods, biogeochemical process measurements, optical methods, or genetic methods. The degree of similarity between these key characteristics of sampled microorganism and known microorganisms can be used to establish identity and infer the physiology, metabolic functions, and ecological traits of the sampled microorganisms by techniques well established in the field of microbial ecology (for example see, Head, et *al.,* 1998; Head, 1999; Gray and Head, 2001; Röling & Head, 2004; Stahl, 1997; Trüper, and Schleifer,1992).

Non-limiting examples of characterization methods that can be used in the process of the invention include:
(a) Enrichment culture techniques to obtain microorganism isolates from which key biochemical, morphological, physiological, ecological, and genetic traits may be determined and compared against the traits of known microorganisms.
(b) Determination of the phospholipid fatty acid composition (PLFA) of the microorganisms and comparison with PLFA distributions of known microorganisms.
(c) Determination of isoprenoid glyceryl ether distributions (archaeols) characteristic of methanogenic or other archaea and comparison with isoprenoid glyceryl ether distributions of known microorganisms.
(d) Genetic characterization methods, of which two non-limiting examples (among many) are listed below:

1. Sequences of genetic fragments from sampled microorganisms including but not restricted to 16S rRNA genes (bacterial, archaeal) genes encoding the alpha subunit of methyl coenzyme A reductase (mcrA)from methanogenic and methane oxidizing archaea and genes encoding the alpha-subunit of benzylsuccinate synthase (*bssA*) and homologues, involved in the initial activation of hydrocarbons by anaerobic hydrocarbon degrading bacteria. These are compared against nucleic acid sequences from known microorganisms (for example, using the Ribosomal Database Project, Michigan State University, East Lansing or the Genbank database at the National Center for Biotechnology Information located in the National Library of Medicine (Building 38A Room 8N805), Bethesda, Md. 20894, U.S.A.) to establish the phylogenetic identity with nearest known relatives using established techniques (Röling & Head, 2004).
   In particular quantitative analysis of these target genes (using real-time PCR)characteristic of particular organisms or processes that must be controlled for maximizing recovery of hydrogen is of use, including but not exclusively dissimilatory sulfite reductase (dsrAB), nitrite reductases (e.g. *nirS* and nirK) and hydrogenases, and for quantification of potential primary hydrocarbon-degrading fermentative syntrophs.
2. Oligonucleotides designed to hybridize to the 16S rRNA genes of Specific microorganisms and target genes indicative of key processes (hydrocarbon activation, methane generation, hydrogen oxidation - see specific examples detailed above) should be used in polymerase chain reaction based methods. Although -potentially applicable the use of such oligonucleotide probes labeled with radioactive phosphorus, biotin, fluorescent dyes, enzymes and other suitable tags are likely to lack the sensitivity required for analysis of subsurface samples unless linked to amplification techniques such as the polymerase chain reaction or culture-based enrichment or analysis of microcosms.

The following paragraphs describe an application of DNA probes to identify the presence and identity of methanogens and methanotrophic archaea which must be inhibited respectively to achieve maximal hydrogen recovery.
(i) Determining the presence and identity of methanogens, sulphate reducers and other hydrogen oxidizing organisms.
   The conversion of petroleum to organic acids and hydrogen is a key step in the syntrophic metabolism of petroleum. Under different conditions different hydrogen-oxidizers will be associated with the syntrophic petroleum degrading organisms. These must be distinguished in order to design the most appropriate interventions to maximise hydrogen production. 16S rRNA genes and genes encoding the alpha subunit of methyl coenzyme M reductase can in principle be used to detect methanogenic archaea. Methyl coenzyme M reductase is found in methanogenic archaea. Homologues of methyl coenzyme M reductase are also found in anaerobic methane oxidizing archaea (Krüger et al., 2003; Hallam et al., 2003) and thus oligonucleotide primers targeting regions which are conserved in methanogen mcrA genes and distinct in methane-oxidizer mcrA genes (Krüger et al., 2003; Hallam et al., 2003) must be designed to distinguish the two types of organism. Alternatively broad speeificity *mcrA* primers must be used (e.g. Lueders and Friedrich, 2003) followed by cloning and sequencing of the mcrA genes sampled in order to determine their provenance. The same principles can be used for different gene targets characteristic of different groups of hydrogen oxidizing microorganisms as described above.

### Determining an ecological environment to stimulate Petroleum Degradation and Hydrogen generation and to retard Hydrogen oxidation

From knowledge of the indigenous microorganisms and their nutritional requirements, the chemical composition of the formation's oil and water and matrix rock, and the physical characteristics of the formation (pressure, temperature, porosity, saturation, etc.), the overall ecological environment needed to promote and retard the activity of the microbial consortium can be determined. This information is then used to modify the formation's environmental parameters to promote microbial conversion of petroleums to hydrogen and to retard microbial degradation of hydrogen.

Altering the activity of microorganisms in the subsurface depends on at least one of the following factors:
1) Adding and/or subtracting and/or maintaining key components required for microbial growth and/or activity as determined by the laboratory and/or in situ pilot studies;
2) Controlling and/or maintaining the subsurface environment (for example, chemistry, temperature, salinity, and pressure); and
3) Specifically inactivating hydrogen consuming organisms either chemically or physically.

### Microbial Ecology

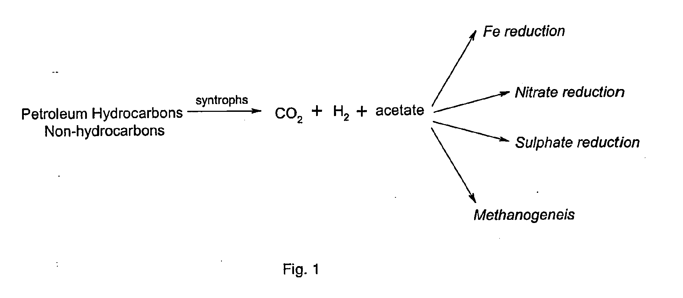

In order to stimulate and/or sustain commercial rates of petroleum degradation and hydrogen generation and to reduce the rate of hydrogen degradation, basic components of the subsurface environment and microbiota are determined. The basic system active in petroleum reservoirs is shown in Fig 1. To accelerate hydrogen production it is necessary to accelerate the fermentative syntrophs while reducing the activity of methanogens, sulphate reducers and other hydrogen-oxidizing organisms coupled to other means of removing the hydrogen generated to a production system.

To convert petroleums to hydrogen, the formation's indigenous microbial consortium may comprise petroleum-degrading microorganisms having similar genetic characteristics to one or more of the microorganisms listed below. Note that if hydrocarbon degrading iron-reducing, nitrate-reducing (including, but not exclusively, denitrifiers) sulphate-reducing bacteria and/or archaea are present specific steps must be taken to inhibit their activity otherwise hydrocarbons will be degraded to carbon dioxide and water (or methane) without the accumulation of hydrogen as an intermediate. Any aerobic hydrocarbon degrading organisms identified are unlikely to be indigenous to the formation. These too however would be detrimental to the process of petroleum hydrocarbon conversion to hydrogen. Such organisms will most likely be inactive unless substantial quantities of oxygen are provided. Potential fermentative, syntrophic organisms that will convert complex organic carbon in petroleum into hydrogen and other fermentation products include organisms related to the following: *Syntrophobacter* spp., *Syntrophus* spp., *Syntrophomonas* spp., *Thermoanaerobacter* and relatives, *Thermotoga, Thermoanaerobacterum, Fervidobacterium, Thermosipho, Haloanaerobium, Acetoanaerobium, Anaerobaculum, Geotoga, Petrotoga, Thermococcus, Pyrococcus, Clostridium* and relatives. Organisms that may result in lower hydrogen yields will also be present in the formation and must be identified in order to design appropriate inhibition strategies. These will primarily be anaerobic hydrogen-oxidizing bacteria and archaea including but not exclusively methanogens and sulphate-reducers.

Understanding the subsurface ecology allows one skilled in the art to deduce likely additives that can stimulate subsurface activity. Additives could include (in an appropriate form for distribution throughout the formation) but are not limited to:
- major nutrients containing nitrogen and phosphorus that do not accelerate competing processes such as nitrate or sulphate reduction, non-limiting examples may include Na₂HPO₄, K₂HPO₄, NH₄Cl added via water injection or ammonia gas (NH₃) or volatile phosphorus (PH₃,CH₃-PH₂) compounds added which can be quickly dispersed through the gas caps facilitating nutrient supply very quickly over large areas of the fields. Phosphates may precipitate chemically in formations and therefore less reactive forms of phosphorus such as polyphosphate and phosphorus pentoxide may be more appropriate additives; NaNO₃, KNO₃, NH₄NO₃, would accelerate syntrophiv hydrogen generation. However addition of nitrate would stimulate nitrate reducing bacteria which would consume hydrogen. Ammoniumion can support subsurface microbial activity and does not facilitate adverse processes such as nitrate reduction. It is therefore vital that the correct form of nitrogen and phosphorus as determined experimentally are added in order that processes that would inhibit hydrogen production are not fortuitously stimulated.

- vitamins (non-limiting examples may include folic acid, ascorbic acid, and riboflavin);
- trace elements (non-limiting examples may include B, Zn, Cu, Co, Mg, Mn, Fe, Mo, W, Ni, and Se);
- buffers for environmental controls; and
- addition of artificial electron acceptors (non-limiting examples may include SO₄²⁻, NO₃²⁻, Fe⁺³, humic acid, mineral oxides, quinone compounds, CO₂, O₂, and combinations thereof) may be added to stimulate microbial activity. However, although these amendments will stimulate microbial activity all will be detrimental to hydrogen generation from petroleum. All of these electron acceptors will stimulate organisms that will efficiently consume hydrogen and thus alter the pathway of petroleum degradation such that hydrogen is not an accumulating intermediate.
- waters of different salinities and pH values or containing complexing agents such as organic acids such as oxalate, EDTA or other multi-dentate ligand organic compounds including but not limited to hydroxylated acids to facilitate mineral dissolution and release of natural nutrients included but not limited to such as ammonium or potassium or phosphate ion from dissolution of feldspars, clays or other silicates and carbonates.

For example, if potassium is known to stimulate growth of the closest-matching syntrophic fermentative microorganism, and if potassium is present in the formation in only limited concentrations in a labile accessible form , then addition of potassium in an accessible soluble form to the formation should also stimulate the uncharacterized fermentative organisms.

Suitable stimulants can be tested and optimized using indigenous microorganisms in laboratory microcosms, cultures or in situ pilot sites to determine their effectiveness at promoting rapid petroleum-degradation. However, any stimulants chosen are also likely to increase the rate of activity of any methanogenic, sulphate reducing or other hydrogen oxidizing microorganisms. Steps must therefore be taken to inactivate or inhibit these organisms.

Indigenous microbial consortia are grown in nutrients using a range of nutrient media, with varying pH, salinity, trace metals, and electron acceptors to find those conditions which support high rates of syntrophic petroleum degradation. Syntrophic petroleum degradation by fermentative bacteria must be linked to hydrogen removal by terminal hydrogen oxidizing organisms. These microcosm and culture studies should be used to identify the major terminal oxidation process and steps taken to inhibit this appropriately (see below) to permit accumulation of hydrogen. These culture studies will typically involve several cycles of stimulant/inhibitor addition and stimulant/inhibitor combinations as well as modified environmental conditions (e.g. salinity, temperature, pH see below). Because the indigenous microorganisms found in a given formation and the chemistry of the formation fluids and formation rocks will typically be unique to that formation, the conditions for promoting growth of indigenous microorganisms may vary from one petroleum accumulation to another and may vary from one location in the petroleum accumulation to another. Conditions favourable for microorganism growth in part of the petroleum accumulation may not be optimum for another part of the petroleum accumulation.

The inventors have concluded that hydrocarbon degradation in deep subsurface petroleum reservoir environments is often phosphorus, potassium or nitrogen limited. In pivotal studies, Bennett and co-workers (Summarised in Bennett et al, 2001; Rogers and Bennett, 2004 and references therein) have shown a close relationship between the geomicrobiology of petroleum-contaminated aquifers, mineral alteration and groundwater chemistry. Biological activity perturbs general groundwater chemistry and therefore mineral-water equilibria, and at the microscale, attached organisms locally perturb mineral-water equilibria, releasing limiting nutrients. In an oil-contaminated aquifer it was shown that feldspars weather exclusively near attached microorganisms in the anoxic region of the contaminant plume and that indigenous bacteria colonized feldspars that contain potassium or trace phosphorus. Most phosphorus in many petroleum reservoir and reservoir encasing sediments is in feldspars and it has been suggested that natural feldspar dissolution in some oil reservoirs (e.g. the Gullfaks field in the North Sea) is related to biodegradation of the associated oils (Ehrenberg and Jacobsen, 2001). Phosphorus contents of oils are low (approximately 1ppm or much less) whereas phosphorus contents of sandstone reservoirs or reservoir encasing shales are much higher (up to 1000ppm or more of oxide equivalents). The phosphorus is thus generally present in mineral phases of low water solubility. Indeed, the inventors believe that supply of limiting nutrients from mineral dissolution in reservoirs or reservoir encasing shales in many instances may be the rate-limiting step in subsurface petroleum biodegradation. Addition of phosphorus as soluble forms of phosphates in injected waters or alteration of reservoir water chemistry by change of pH, salinity or addition of complexing agents including organic acids or multi dentate organic chelating agents can be used to release available phosphorus or potassium to accelerate petroleum biodegradation. Ammonium phosphate or potassium ammonium phosphate would add both essential nitrogen and phosphate and also potassium.

The present inventors have determined that concentration of ammonium ion (NH₄⁺) in the formation waters is also critical to the rate of biodegradation. Naturally in petroleum reservoirs mean concentrations of ammonium ion range from a few ppm up to a up around 500 ppm but are typically around a few tens of ppm (Manning and Hutcheon, 2004). In contrast in near surface anoxic environments (e.g. landfills) concentrations of ammonium ion range up to over 1000ppm. Nitrogen supplied in the form of ammonium ion will accelerate biodegradation whereas if supplied as nitrate, nitrate reduction will eliminate or reduce hydrogen production.

In sandstone reservoirs, reservoirs in which petroleum is trapped in the pore systems of sandstones, the present inventors have determined that the concentration of nutrients such as phosphorous is rate limiting on overall oil biodegradation rate and thus hydrogen production. The concentration of phosphorous may be increased by the addition of exogenous phosphorous, or by release of phosphorous from the reservoir matrix by modifying the characteristics of the reservoir waters such that the phosphorus containing minerals in the reservoir such as silicates or carbonates dissolve releasing their phosphorus. For example injection of fresh, low salinity waters or acidic waters will aid in feldspar dissolution releasing nutrients. Addition of organic acids such as oxalate, EDTA, citrate or other multi-ligand chelating agents including hydroxylated acids and other multi functional chelating agents would facilitate mineral dissolution and release of natural phosphorus and other essential nutrients from reservoir minerals. These treatments may stimulate all microorganims present, not only those required for conversion of petroleum to hydrogen. To prevent the activity of organisms that will consume any hydrogen generated certain amendements or physical treatments may be required to suppress their activity. These may include (but are not exclusive to) sodium molybdate (or other hexavalent cation) to inhibit sulphate-reducing bacteria and sodium chlorate to inhibit nitrate-reducing bacteria. Methanogens can be inhibited with bromoethane sulfonic acid, N-substituted derivatives of paraaminobenzoic acid and several other compounds.

In addition a number of fermentative syntrophic microorganisms form spores whereas most of the terminal hydrogen-oxidizing organisms do not and are hence more temperature sensitive. Selection of low temperature reservoirs that have previously been exposed to higher temperatures (palaeopasteurized; Wilhelms et al., 2001) or artificially pasteurizing low temperature reservoirs by steam injection for example and then stimulating syntroph activity may be used to promote fermentation reactions while inactivating hydrogen-oxidizing components of the microbial consortium present. Sterile reservoirs may be inoculated with exogenous fermentative syntrophic organisms that generate hydrogen.

### Formation Conditions

Environmental conditions in petroleum bearing, subterranean formations may not be conducive to thriving populations of the appropriate indigenous microorganisms. The appropriate microorganisms may need to be stimulated to be more active. This stimulation is carried out by modifying one or more parameters of the formation environment. For example, high-salinity environments may greatly slow the rates of petroleum degradation. Introduction of low salinity waters may stimulate the degradation and hydrogen formation.

Equally, the environment may also be altered to slow the rate of hydrogen degradation. Ideally, the changes required to increase the rates of petroleum degradation or hydrogen production will simultaneously decrease the rate of hydrogen degradation.

The present invention can be practiced in any petroleum-bearing formation that is suitable for microbial life or that can be modified to be suitable for microbial life. In general, the formation fluids will have a temperature less than about 135°C, a pressure less than about 10,000 psig (6895 kPa), a subsurface pH between about 3 and 10, and a salt concentration less than about 300,000 parts per million. Reservoirs cooler than 80 degrees centigrade or which can be cooled to below 80 centigrade are the optimal reservoirs for treatment. The inventors have shown that indigenous organisms are not likely to be active in reservoirs hotter than 80°C or where geochemical and geological data indicate the reservoir has ever been heated to more than 80°C (Wilhelms et al, 2001). In these circumstances injection of exogenous hydrogen generating consortia will be necessary.

Formation environmental parameters of principal concern for providing optimal petroleum degradation and hydrogen generation conditions include, but are not limited to, temperature, salinity, pH, alkalinity, organic acid concentration, nutrients, vitamins, trace elements, availability of terminal electron acceptors (high levels will suppress hydrogen generation), and toxic substances (to suppress the activity of competing microorganisms). One or more of these environmental parameters may require adjustment or maintenance within specific ranges to initiate or sustain commercial rates of hydrogen generation.

The environmental conditions for promoting growth of a microbial consortium in a formation will necessarily involve many factors including, without limiting the scope of this invention, the following, either alone or in combination:
● changes in the formation temperature, pH, Eh, mineralogy, and salinity and the concentrations of CO₂, O₂, and H₂ in the formation; and
● creation, movement and/or maintenance of boundaries, sometimes referred to as "environmental interfaces", between different petroleum-degradation microbial populations, and/or microbial hydrogen generating zones.

### Modifying the Formation Environment (Adding Stimulants, inhibitors and/or Changing Environmental Factors)

The additions of stimulant(s), inhibitor(s) or change(s) of environmental factor(s), either alone or in combination, are referred to in this description as microbial growth "modifiers". The particular modifier, or combination of modifiers, suitable for a particular application will depend on the microbial consortium to be modified and the formation environmental conditions. Since certain indigenous microorganisms are typically in a nutrient deprived state, one stimulation strategy will typically involve addition of nutrients. However, since stimulating hydrogen production is also likely to stimulate hydrogen degradation, the modifier package will often contain an inhibitor of hydrogen-oxidizing organisms (see comments above). Once a modifier package is determined, the formation environment can be altered on a continuing basis or discontinued after a suitable period of time to permit change in the populations of the microorganisms.

### Injection Process

For growth or activity modifiers that involve injecting a material into the formation, the material can be added to a fluid flood such as an aqueous solution or gas (such as CO₂) or solvent or polymer that is injected into the formation by any procedure found most convenient and the invention is not limited to any particular process of introducing the stimulants. The implementation of the present invention will often involve adding the stimulant package to a waterflood program. To simplify the following discussion, the above-identified injection carrier will be referred to as water.

Formation stimulants can be added to water and injected into the formation through one or more injection wells and pressured to flow toward one or more production wells. Underground oil formations are frequently flooded with water in order to supply additional pressure to assist oil recovery. The microbial stimulant is preferably injected into a well as part of the injection program of the waterflood.

The amount of water introduced into the formation and the amounts of microbial modifiers contained in the water will depend upon the results desired. Those skilled in the art can determine the amount needed to provide hydrogen production based on the information in this description.

Multiple modifiers can be injected into the formation together or in separate injection steps. For example, a slug or bank of water carrying one modifier may be followed by a second bank or slug of water carrying a second modifier package. Another example may include injecting one water bank followed by a gas injection step, sometimes referred to as a WAG process. Injection of gas below the degrading oil column may facilitate circulation of waters and nutrients to the microorganisms and may also allow for injection of soluble or volatile microbially accessible nutrients which would disperse rapidly in the reservoir environment.

Layered reservoir bioreactors are the most feasible to implement for hydrogen production and facilitated hydrogen removal. In such a reservoir bioreactor, the biodegrading oil column and/ or residual oil zones could be vertically segmented and the environments could be controlled in the following manner:
(a) A lower zone of degradation of oil or injected reactive organic substrates is environmentally modified to produce abundant free gas-usually methane but possibly methane and carbon dioxide.
(b) An upper zone of degradation of oil or injected reactive organic substrates is environmentally modified to produce abundant free hydrogen.
(c) Free gas or gas charged water from the lower layer buoyantly moves up through the layered reservoir bioreactor and any free hydrogen or hydrogen in aqueous or oil solution partitions into the moving fluids and is carried to the gas cap for production.

Gas sparging or flushing of degrading oil columns by injecting gas from a well or by producing gas in a biodegrading reservoir layer below the zone to be flushed could also be employed. A gas phase (methane, carbon dioxide, air) could be injected below the degrading oil column. With methane and carbon dioxide simple partitioning would occur and remove hydrogen as a free gas phase. With air, aerobic degradation of organic matter at the base of the column would facilitate pressure production and large volumes of gas(carbon dioxide) to carry up into an anaerobic zone where hydrogen production was occurring.

Gas sparging or flushing of degrading oil or residual oil zones would also facilitate introduction of nutrients either as entrained water soluble nutrients or via volatile gas transported nutrients. This would be a very fast way of getting nitrogen, phosphorous and other nutrients to the hydrogen production zones.

Gas sparged reservoirs or reservoirs operating without gas sparging ideally would have injector wells below the initial oil water contact (owc) to inject nutrients, inhibitors and metabolic modifiers.

Gas production would be from conventional production wells producing either free gases or gases dissolved in fluids-water or oil. Hydrogen could be separated from the produced gases by a variety of separation schemes including membranes or other physical separation systems. Environmentally unfriendly gases such as carbon dioxide could be reinjected to the formations below.

Production of hydrogen would be aided by production plans designed using reservoir simulators than can simulate gas generation and gas and oil migration within reservoirs.

### Creation/Maintenance of Environmental Interfaces

Microorganisms in subterranean formations tend to be most active at environmental boundaries such as between fermentation zones and methanogenesis zones. Therefore, microorganism activity in a formation may be increased by increasing the number of such boundaries, which serve as environmental interfaces. US 6,543,535 claims one method for increasing the number of environmental interfaces is to modify the water flood injection rates. A second method is to alternate or vary the injection modifiers into the formation to in effect create moving environmental fronts. A third method involves forming small-scale environmental interfaces by forming petroleum-water emulsions in the formation or by changing the clay chemistry. The present inventors consider that a very practical fourth method relies on knowledge of field geometry and knowledge of the relationships of oil and water layers naturally distributed. The optimal fields for processing for hydrogen production are fields where already existing existing natural interfaces between water and oil are large. These include any fields with residual oil columns produced either naturally over geological time or via primary or enhanced recovery procedures.

The most optimal fields for recovery of oil as hydrogen would be those fields that have large residual oil columns below the producible oil legs.

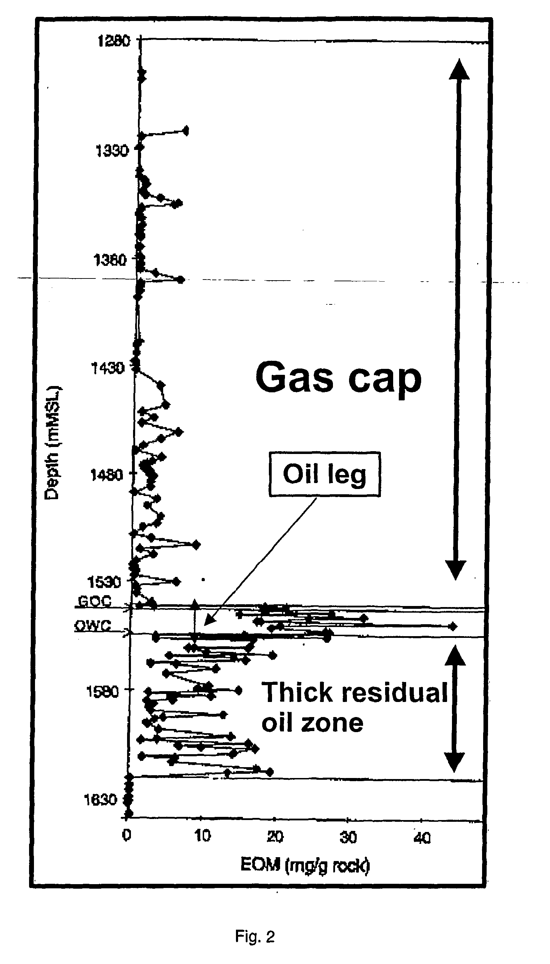

Though not limited by field configuratinon, the most optimal fields for recovery of oil as hydrogen would be those fields that have large residual oil columns below the producible oil legs. A common process during field filling is movement of oil legs through field tilting, leakage of oil through seals and during the biodegradation process oil is naturally consumed and oil legs move upwards leaving a residual oil zone with large water/oil interfacial areas. The inventors have determined that the best fields for recovery of oil as hydrogen such as the Troll field or Frigg field in the N.Sea often have have thick residual oil zones ideal for processing to hydrogen through microbial activity (Horstad and Larter, 1997; Larter et al, 1999).(Fig 2) These fields are ideally produced without sulphate injection in seawater using other schemes such as gas cap expansion or natural depletion.

Figure 2 shows an ideal configuration for a field to produce hydrogen and recover both residual oil and producible oil as hydrogen. The figure shows the oil yield as a function of depth in the reservoir for a large oil and gas field in the North Sea (Troll field-after Horstad and Larter, 1997). Gas production from the gas cap would recover gas which is partly derived from microbial conversion of oil in the oil leg and residual oil zone to hydrogen. As oil is produced from the oil leg water moves up into the residual oil zone and oil leg facilitating petroleum biodegradation and oil recovery as hydrogen by increase of oil/water surface area and addition of nutrients, metabolic modifiers or organisms under the oil leg which can migrate up through the reacting petroleum column to accelerate hydrogen generation processes and retard hydrogen destruction processes.

Such high interfacial area zones can also be produce through normal recovery processes as oil legs are produced to leave a residual oil zone. Such dispersed oil zones are ideal for promotion of microbial activity as the water/oil interface is large facilitating easy transmission of nutrients, metabolic modifiers or organisms to the reaction sites in the oil leg.

### Changing Environmental Conditions

Changing the environmental conditions for promoting growth of the microbial consortium in a formation can be accomplished by injection of material into the formation. Environmental factors that can be changed include formation temperature, pH, Eh, and salinity and the concentrations of CO₂, O₂, and H₂ as well as other electron donors and acceptors. As discussed above, the most likely process of environmental change will be by injection of fluids (e.g. water, solvent, and polymer) or gases as part of the secondary or tertiary recovery process.

The ideal location of injection wells is below the current oil water contacts or residual oil zones that migrate upwards during normal oil production or consumption of oil during biodegradation that allows the oil zone to move upwards facilitating movement of water through any residual oil remaining. This allows for modifying agents to be dispersed upwards into the remaining oils facilitating increased degradation rates and hydrogen production.

As an example of changing environmental conditions, oil formation waters often contain low concentrations of indigenous phosphate ion which the inventors consider to be a rate controlling nutrient in most biodegrading reservoirs. Injecting water of very low salinity or with a pH different from the formation pH or waters containing organic acids such as oxalate or citrate or other complexing agents including multidentate chelating agents would aid in the dissolution and release from minerals such as feldspars or clays key nutrients such as phosphorous, nitrogen, potassium, cobalt or nickel. Alternatively phosphorus could be added as phosphate, polyphosphate or phosphorus pentoxide, nitrogen as ammonium ion or urea and potassium, cobalt or nickel as water soluble salts.

### Monitoring the Process

During the injection process for stimulating microbial transformation of petroleums to hydrogen and inhibiting microbial degradation of hydrogen, both the formation conditions and the microbial dynamics (ecology) are preferably monitored. This monitoring can be performed in any suitable manner. Normally fluid (for example, oil, gas, and water) samples will be obtained from the formation through one or more wells in communication with the formation. The samples are analyzed to determine the concentration and type of microorganisms in the fluid as well as the concentration of modifiers and microbial products in the fluid. Other geochemical analyses may also be performed to assess the effectiveness of the stimulants on the formation environment and to confirm the chemical compatibility of the desired injectant and the subsurface fluids and solids. If based on this monitoring the modifier effect in the formation is outside the desired range, the concentration of modifier in the waterflood may be adjusted accordingly to bring the modifier effect back within acceptable range to optimise hydrogen production.

### Production

Recovery of hydrogen produced by the microbial activity can be by any suitable gas production technology. The described process is not in any way restricted to secondary or tertiary oil recovery. The process can be used simultaneously with injection of water in secondary oil recovery, at the end of secondary recovery, or at the start of production of an oil field if and when injection of water is found feasible. After introduction of the stimulant package into the formation, the formation may-be-shut in for a sufficient period of time to allow the microorganisms to produce hydrogen or production may be maintained throughout. The hydrogen may accumulate in a gas zone or gas cap, a free-gas phase overlying a petroleum zone or as an enhanced hydrogen concentration within the original petroleum phase. This gas may be withdrawn through a conventional gas production well that communicates with the gas zone or gas cap. In other formations, the gas may be produced as a product entrained in produced oil and water. In still other formations, the gas may be produced through different zones of wells previously used in production of liquid petroleums from the formation. To enhance microbial gas exsolution (release) from unrecoverable oil and subsequent gas production, it may be beneficial to drop the overall formation pressure by water well production. This invention is not limited by the technology used to recover the hydrogen. Biodegrading reservoirs allow novel forms of gas recovery. Layered reservoir bioreactors are discussed above.

Acceleration of hydrogen generation, provision of nutrients, injection of organic matter degrading microorganisms and production of gases (methane and carbon dioxide) can be facilitated by injection of reactive organic matter into or below biodegrading petroleum columns. Organic matter may be from sewage, waste waters, biomass and industrial chemical wastes and farm wastes among others. Such materials could be injected as part of a normal reservoir pressure maintenance program into actively degrading petroleum columns or into sterile columns needing inoculation with organic matter degrading organisms.

While microorganisms can be injected into a reservoir formation microorganisms naturally present in the formation are preferred because it is known that they are capable of surviving and thriving in the formation environment. Indeed the inventors consider that the fields most favourable for petroleum recovery as hydrogen are fields that are currently actively biodegrading. However, this invention is not limited to use of indigenous microorganisms. Exogenous microorganisms suitable for growing in the subterranean formation may be introduced into the formation by known injection techniques before, during, or after practicing the process of this invention.

The following field example illustrates a specific actual embodiment of the invention.

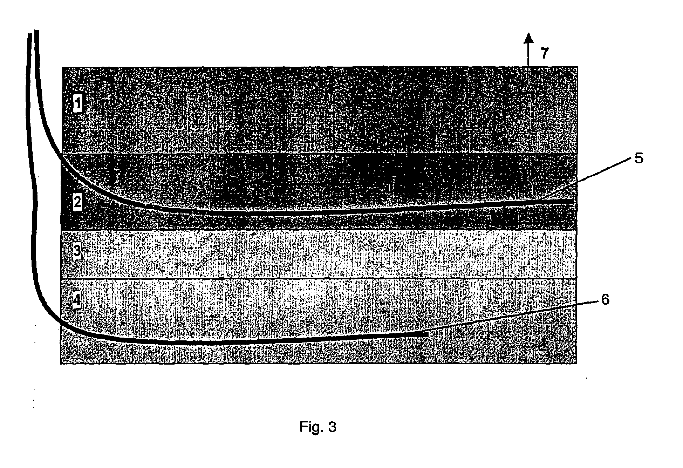

For this hypothetical example, reference is made to Figure 3, which illustrates a horizontal production or injection will 5 in a field with a mobile producible oil leg 2 and a residual oil zone 3 below it with a water leg 4 below that. The oil leg 2 is overlain by a producible gas cap 1. The reservoir shows indications of active indigenous microorganisms. A horizontal injector well 6 underlies the petroleum accumulation in the water leg 4. Oil production initially occurs from the upper production well 5 allowing water to migrate up through the residual oil zone 3 below the oil column 2. To facilitate hydrogen production by acceleration of the indigenous microorganisms in the upper part of the residual oil zone and the oil leg, water containing one or more stimulants or adverse process suppressants may be injected through the upper well 5 or injected through the lower injection well 6.

As the subsurface microbes increase the conversion of oil in the pores to hydrogen, the hydrogen concentration (not shown) increases in the fluid phases (water and oil). Eventually the hydrogen concentration may exceed the saturation level in the fluids and form bubbles of hydrogen. The generated hydrogen can migrate to the top of the formation to add to the existing gas cap 1 which is under production 7 or flow as dissolved gas in oil produced at an oil production well 5. The hydrogen can for example be dissolved in oil in the mobile oil zone or dissolved in produced water. The hydrogen can also flow as a separate gas phase along with produced oil and water. The hydrogen is recovered at a production well along with produced oil and water. As oil and gas is produced the waters containing any injected stimulants or suppressants rises through the residual oil zone facilitating further accelerated conversion of oil to hydrogen. Injection of fluids or fluid organic wastes such as sewage into the injector well below the petroleum column would introduce microorganisms, nutrients and reactive organic matter which would produce abundant gas (methane, carbon dioxide and possibly some hydrogen), increase in formation pressure improving oil recovery and produce gas bubbles which would aid in movement of waters up through the oil zones transporting nutrients and help transport hydrogen through to the gas cap or oil leg where it can be produced in conventional production wells. Gas dissolving in the oil would decrease its viscosity and this together with any increase in pressure would facilitate any continuing oil recovery process in addition to any hydrogen production.

In the example shown export of hydrogen from the zone of enhanced syntrophic bacterial activity in the upper residual oil zone and oil leg is facilitated by methane and carbon dioxide flushing from the degradation zone in the lower residual oil zone below the hydrogen generating zones or from gases injected into the lower injector well 6. This hydrogen removal facilitates increased extent and rate of hydrogen production.

### References

### References

Aitken, C.M, Jones,D.M. & Larter,S.R. 2002, Isolation and identification of biomarkers indicative of anaerobic biodegradation in petroleum reservoirs., Abstracts of the 2002 William Smith Meeting, Geological Society of London, October, 2002. Full article in final review in Nature.
Anderson, RT & Lovley, DR. 2000. Hexadecane decay by methanogenesis, Nature, 404, 722-723.
Annweiler, E., Michaelis, W. and Meckenstock, R.U. Identical Ring Cleavage Products during Anaerobic Degradation of Naphthalene, 2-Methylnaphthalene, and Tetralin Indicate a New Metabolic Pathway. Applied and Environmental Microbiology, 68, 852-858 (2002).
Beller, H.R. (2002). Analysis of Benzylsuccinates in Groundwater by Liquid Chromatography /Tandem Mass Spectrometry and Its Use for Monitoring In Situ BTEX Biodegradation," Environmental Science and Technology 36, 2724-2728.
Bernard, F.P. and Connan, J., Indigenous microorganisms in connate waters of many oilfields: A new tool in exploration and production techniques. 67th Annual technical conference and exhibition of the Society of Petroleum Engineers, Vol. SPE 24811, Washington, DC, 1992, pp. 467-476.
Bennett, P.C, Rogers, JR & Choi, WJ, 2001. Silicates, silicate weathering, and microbial ecology., Geomicrobiol. J., 18, 3-19. Boreham,C.J. Hope, J.M. & Hartung-Kagi, B. 2001. Understanding source, distribution and preservation of Australian natural gas: A geochemical perspective. APPEA Journal 41, 523-547.
Connan, J. 1984. in Advances in Petroleum Geochemistry, Vol. 1 (eds Brooks, J. & Welte, D. H.) 299-335. (Academic Press, London.)).
Dessort, D., Poirier, Y., Sermondadez, G. & Levache, D. 2003. Methane generation during biodegradation of crude oil. Abstracts of the 21st IMOG. Krakow, Poland, September 2003.
Ehrenberg SN, Jakobsen KG, Plagioclase dissolution related to biodegradation of oil in Brent Group sandstones (Middle Jurassic) of Gullfaks Field, northern North Sea. SEDIMENTOLOGY 48 (4): 703-721 AUG 2001
Gray, N.D. & Head, I.M. (2001). Linking genetic identity and function in communities of uncultured bacteria. Environmental Microbiology 3, 481-492
Hallam, S.J. , Girguis,P.R., Preston, C. M., Richardson, P.M. and DeLong, E.F. (2003). Identification of methyl coenzyme M reductase a (mcrA) genes associated with methane-oxidizing archaea. Applied and Environmental Microbiology, 69, 5483-5491.
Head, I.M., Saunders, J.R. & Pickup, R.W. (1998). Microbial evolution, diversity and ecology: a decade of ribosomal RNA analysis of uncultured microorganisms. Microbial Ecology 35, 1-21
Head, I.M. (1999). Recovery and analysis of ribosomal RNA sequences from the environment. p. 139-174. In: Environmental Monitoring of Bacteria. edited by C. Edwards. Methods in Biotechnology Volume 12. Humana Press, Totowa, New Jersey
Horstad, I. and Larter, S.R., Petroleum migration, alteration, and remigration within Troll field, Norwegian North Sea, Aapg Bulletin-American Association of Petroleum Geologists, 81 (1997) 222-248.
Head, I., Jones, D.M. & Larter,S.R. 2003. Biological activity in the deep subsurface and the origin of heavy oil. Nature, 426, 344-352.
James, A.T. & Burns, B.J. (1984) Microbial alteration of subsurface natural gas accumulations. AAPG Bulletin 68, 957-960.
Krüger, M., Meyerdierks, A., Glöckner, F.O., Amann, R., Widdel1, F., Kube, M., Reinhardt, R., Kahnt, J., Böcher, R., Thauer, R.K & Shima, S. 2003. A conspicuous nickel protein in microbial mats that oxidize methane anaerobically. Nature 426, 878-881
Larter, S., Hockey, A., Aplin, A., Telnaes, N., Wilhelms, A., Horstad, I., Di Primio, R. and O., S., When biodegradation preserves petroleum! Petroleum geochemistry of N. Sea Oil Rimmed Gas Accumulations (ORGA's). Proceedings AAPG Hedberg Research Conference on "Natural Gas Formation and Occurrence", Durango, Colorado, 1999, pp. 3.
Larter, S.R., Wilhelms, A., Head, I., Koopmans,M, Aplin,A., Di Primio,R, Zwach, C., Erdmann,M. and Telnaes, N.(2003) The controls on the composition of biodegraded oils in the deep subsurface:(Part 1) Biodegradation rates in petroleum reservoirs, Organic Geochemistry, V34, 601-613(2003)
Lueders, T. and Friedrich, M.W. (2003). Evaluation of PCR amplification bias by terminal restriction fragment length polymorphism analysis of small-subunit rRNA and mcrA genes by using defined template mixtures of methanogenic pure cultures and soil DNA extracts. Applied and Environmental Microbiology, 69, 320-326.
Magot, M., Ollivier, B. & Patel, B.K.C. 2000, Microbiology of petroleum reservoirs, Antonie Van Leeuwenhoek International Journal of General and Molecular Microbiology, 77, 103-116.
Manning, D.A.C. and Hutcheon, I.E. Distribution and mineralogical controls on ammonium in deep groundwaters. Applied geochemistry, in press.
Masterson, W.D., et al. 2001. Evidence for biodegradation and evaporative fractionation in West Sak, Kuparuk and Prudhoe Bay field areas, North Slope, Alaska, Org. Geochem. 32, 411-441.
Mueller, R.F. & Nielsen, P.H. 1996. Characterization of thermophilic consortia from two souring oil reservoirs, Applied and Environmental Microbiology, 62, 3083-3087.
Nazina, T.N., Ivanova, A.E., Borzenkov, I.A., Belyaev, S.S. & Ivanov, M.V. 1995. Occurrence and geochemical activity of microorganisms in high- temperature, water-flooded oil fields of Kazakhstan and Western Siberia, Geomicrobiology Journal, 13, 181-192.
Nazina, T.N., Ivanova, A.E., Golubeva, O.V., Ibatullin, R.R., Belyaev, S.S. & Ivanov, M.V., Occurrence of Sulfate-Reducing and Iron-Reducing Bacteria in Stratal Waters of the Romashkinskoe Oil-Field, Microbiology. 1995, 64, 203-208.
Ng, T.K., Weimer, P.J. & Gawel, L.J. 1989. Possible Nonanthropogenic Origin of 2 Methanogenic Isolates From Oil-Producing Wells in the San-Miguelito Field, Ventura County, California, Geomicrobiology Journal, 7, 185-192.
Nilsen, R. K. & Torsvik, T. 1996. Methanococcus thermolithotrophicus Isolated from north sea oil field reservoir water. Appl. Environ. Microbiol. 62, 1793-1798.
Orphan, V.J., Taylor, L.T., Hafenbradl, D. & Delong, E.F. 2000. Culture-dependent and culture-independent characterization of microbial assemblages associated with high temperature petroleum reservoirs, Applied and Environmental Microbiology, 66, 700-711.
Obraztsova, A.Y., Shipin, O.V., Bezrukova, L.V. and Belyaev, S.S., Properties of the Coccoid Methylotrophic Methanogen, Methanococcoides-Euhalobius Sp-Nov, Microbiology, 56 (1987) 523-527.
Pallasser, R.J. 2000. recognising biodegradation in gas/oil accumulations through the del13C composition of gas components., Org. Geochem., 31, 12, 1363-1373.
Rabus, R., Wilkes, H., Behrends, A., Armstruff, A., Fischer, T., Pierik, A.J. and Widdel, F. Anaerobic Initial Reaction of n-Alkanes in a Denitrifying Bacterium: Evidence for (1-Methylpentyl) succinate as Initial Product and for Involvement of an Organic Radical in n-Hexane Metabolism. Journal of Bacteriology, 183, 1707-1715 (2001).
Rogers JR, Bennett PC Mineral stimulation of subsurface microorganisms: release of limiting nutrients from silicates, CHEMICAL GEOLOGY ,203 (1-2): 91-108 JAN 15 2004
Röling, W.F.M., & Head I.M. (2004). Prokaryotic systematics: PCR and sequence analysis of amplified 16S rRNA genes. Advanced Methods in Molecular Microbial Ecology, Biosci Publishers.
Röling W.F.M., Head, ,I.M., & Larter, S.R. 2003. The microbiology of hydrocarbon degradation in subsurface petroleum reservoirs: perspectives and prospects, Research in Microbiology, 154, 321-328.
Rozanova, E.P., Savvichev, A.S., Karavaiko, S.G. & Miller, Y.M. 1995. Microbial Processes in the Savuiskoe Oil-Field in the Ob Region, Microbiology, 64, 85-90.
Scott, A.R., Kaiser, W.R. & Ayers, W.B.J. 1994. Thermogenic and secondary biogenic gases, San-Juan Basin, Colorado and New Mexico - Implications for coalbed gas producibility, Bulletin of the American Association of Petroleum Geologists, 78,1186-1209.
Stahl, D.A., (1997) Molecular Approaches for the measurement of density, diversity, and phylogeny. In: Manual of Environmental Microbiology (editors C. J. Hurst; G. R. Knudsen, M. J. McInerney, L. D. Stetzenbach, M. V. Walker), ASM press, Washington D.C., 1997, pp. 102-114.
Sweeney, R.E. & Taylor, P., 1999. Biogenic methane derived from biodegradation of petroleum under environmental conditions and in oil & gas reservoirs. In: Schoell, M., and Claypool, G.E., (Eds.), Proceedings of the AAPG Hedberg Research Conference, 6-10 June, 1999.
Truper, H. G. and Schleifer, K-H. (1992). Prokaryote Characterization and Identification. In: The Prokaryotes, Second Edition, (eds., A. Balows, H. G. Truper, M. Dworkin, W. Harder, K-H. Schleifer) 1992 Vol 1, pp. 126-148.
Wenger, L.M., Davis, C.L. & Isaksen, G.H. 2001. Multiple controls on petroleum biodegradation and impact in oil quality., SPE 71450, Society of Petroleum Engineers, 2001.
Widdel, F. & Rabus, R. 2001. Anaerobic biodegradation of saturated and aromatic hydrocarbons, Current Opinion in Biotechnology, 12, 259-276.
Wilhelms, A., Larter, S.R., Head, I., Farrimond, P., di-Primio R. & Zwach, C. 2001. Biodegradation of oil in uplifted basins prevented by deep-burial sterilisation., Nature 411, 1034-1037.
Wilkes, H., Rabus, R., Fischer, Th., Armstroff, A., Behrends, A. & Widdel, F. 2002. Anaerobic degradation of n-hexane in a denitrifying bacterium: Further degradation of the initial intermediate(1-methylpentyl) succinate via C-skeleton rearrangement. Archives of Microbiology, 177 (3): 235-243
Zengler, K., Richnow, H. H., Rossella-Mora, R., Michaelis, W. & Widdel, F. 1999. Methane formation from long-chain alkanes by anaerobic microorganisms. Nature 401, 266-269.

## Claims

1. A process for stimulating microbial hydrogen production in a petroleum-bearing subterranean formation in which a microbial consortium comprising one or more hydrogen-consuming organisms is present, comprising:
(a) analyzing one or more components of the formation to determine characteristics of the formation environment;
(b) detecting the presence of a microbial consortium, comprising at least one fermentative syntrophic microorganism, within the formation;
(c) assessing whether the formation microrganisms are currently active;
(d) determining whether the microbial consortium comprises one or more fermentative syntrophic microorganisms;
(e) characterization of one or more fermentative syntrophic microorganism of the consortium, and comparing the one or more **characterized** organisms with at least one known **characterized** known microorganism having one or more known physiological and ecological characteristics;
(f) characterizations of said one or more hydrogen-consuming microorganisms, such as methanogens, sulphate reducers, or other hydrogen oxidizing organisms of the consortium, and comparing the one or more **characterized** microorganims with at least one known **characterized**, known microorganism having one or more known physiological and ecological characteristics;
(g) using information obtained from steps (a) through (e) for determining an ecological environment that promotes in situ microbial degradation of petroleum and promotes microbial generation of hydrogen by at least one fermentative syntrophic microorganism of the consortium;
(h) using information obtained from steps (a) and (f) for determining an ecological environment that inhibits in situ microbial degradation of hydrogen by said at least one hydrogen-oxidizing microorganism of the consortium;
(i) modifying the formation environment based on the determinations of step (g) and (h) to stimulate microbial conversion of petroleum to hydrogen and inhibit microbial conversion of hydrogen to other compounds; and
(j) removal of the hydrogen generated from the sites of generation, so as to maintain an energetically feasible microbial conversion of petroleum to hydrogen.

2. A process according to claim 1, wherein the removal of hydrogen may be accomplished by using purge or sparge gases generated or injected into the reservoir.

3. A process according to either claim 1 or claim 2, wherein the step of detecting the presence of anaerobic hydrocarbon-degrading bacteria is part of step (b).

4. A process according to any one of claims 1 to 3, which includes identifying by-products of indigenous microbial activity.

5. A process according to claim 4, wherein the products identified include anaerobic hydrocarbon degradation metabolites.

6. A process according to any one of claims 1 to 3, which includes identifying archaeols.

7. A process according to any one of claims 1 to 6, wherein the analysis in step (a) is focussed on the oil-water transition zones in the formation.

8. A process according to claim 1, wherein geochemical proxies are used to assess whether the formation is actively degrading.

9. A process according to any one of claims 1 to 8, wherein steps (e) and (f) include characterisation of the micororganisms using genetic characterisation methods.

10. A process according to claim 9, wherein the genetic characterisation methods include comparison of sequences of genetic fragments sampled from the microorganisms against sequences from known microorganisms.

11. A process according to any one of claims 1 to 10, wherein the step of modifying the formation environment includes introducing an additive selected from:
(a) major nutrients;
(b) vitamins;
(c) trace elements;
(d) buffers;
(e) waters of:
(i) different salinities;
(ii) different pH values;
(iii) containing complexing agents;
(f) inhibitors of hydrogen-oxidising organisms.

12. A process according to claim 11, wherein the formation environment is modified by additing phosphorus.

13. A process according to claim 11, the formation environment is modified by adding ammonium ion.

14. A process according to any one of claims 1 to 13, wherein the step of modifying the formation environment includes gas sparging.

15. A process according to any one of claims 1 to 14, wherein hydrogen production is promoted by the use of a layered reservoir configuration whereby hydrogen generation in an upper reservoir layer is maintained and promoted by hydrogen removal using gas generated or injected into a lower reservoir layer.

16. A process according to any one of claims 1 to 15, wherein the step of modifying the formation environment includes injection of reactive liquid organic matter into the formation.

## Patentansprüche

1. Verfahren zum Stimulieren von mikrobieller Wasserstoffproduktion in einer unterirdischen Formation, die Erdöl enthält und in der eine Gruppe von Mikroben existiert, die einen oder mehrere Wasserstoff verbrauchende Organismen umfasst, wobei das Verfahren Folgendes umfasst:
(a) das Analysieren einer oder mehrerer Komponenten der Formation zur Bestimmung von Eigenschaften des Umfelds der Formation;
(b) das Detektieren des Vorhandenseins einer Gruppe von Mikroben, die zumindest einen syntrophen Gärungsmikroorganismus umfasst, in der Formation;
(c) das Bestimmen, ob die Mikroorganismen in der Formation gerade aktiv sind;
(d) das Feststellen, ob die Gruppe von Mikroben einen oder mehrere syntrophe Gärungsmikroorganismen umfasst;
(e) das Charakterisieren eines oder mehrerer syntropher Gärungsmikroorganismen der Gruppe und das Vergleichen des einen oder der mehreren charakterisierten Organismen mit zumindest einem bekannten, charakterisierten Mikroorganismus, der eine oder mehrere bekannte physiologische und ökologische Eigenschaften aufweist;
(f) das Charakterisieren eines oder mehrerer Wasserstoff verbrauchender Mikroorganismen, wie z.B. von methanogenen, Sulfat reduzierenden oder anderen Wasserstoff oxidierenden Organismen, der Gruppe und das Vergleichen des einen oder der mehreren charakterisierten Mikroorganismen mit zumindest einem charakterisierten, bekannten Mikroorganismus mit einer oder mehreren bekannten physiologischen und ökologischen Eigenschaften;
(g) die Verwendung der in den Schritten (a) bis (e) gewonnenen Informationen zur Bestimmung eines ökologischen Umfelds, das einen mikrobiellen Abbau von Erdöl in situ und die mikrobielle Produktion von Wasserstoff durch zumindest einen syntrophen Gärungsmikroorganismus der Gruppe begünstigt;
(h) die Verwendung der in den Schritten (a) und (f) gewonnenen Informationen zur Bestimmung eines ökologischen Umfelds, das den mikrobiellen Abbau von Wasserstoff in situ durch den zumindest einen syntrophen Gärungsmikroorganismus der Gruppe verringert;
(i) das Modifizieren des Umfelds der Formation auf Grundlage der Erkenntnisse aus den Schritten (g) und (h) zum Stimulieren des mikrobiellen Abbaus von Erdöl zu Wasserstoff unter Hemmung der mikrobiellen Umwandlung von Wasserstoff in andere Verbindungen; und
(j) das Entfernen des produzierten Wasserstoffs aus den Produktionsstätten, um eine energetisch durchführbare mikrobielle Umwandlung von Erdöl in Wasser aufrechtzuerhalten.

2. Verfahren nach Anspruch 1, worin die Entfernung von Wasserstoff unter Einsatz von Spülgas oder verteiltem Gas erfolgen kann, das in dem Reservoir produziert wird oder in dieses eingeleitet wird.

3. Verfahren nach Anspruch 1 oder 2, worin der Schritt der Detektion des Vorhandenseins von anaeroben ölzersetzenden Bakterien Teil von Schritt (b) ist.

4. Verfahren nach Anspruch 1 bis 3, das das Identifizieren von Nebenprodukten indigener mikrobieller Aktivität umfasst.

5. Verfahren nach Anspruch 4, worin die identifizierten Produkte anaerobe Kohlenwasserstoffabbau-Metaboliten umfassen.

6. Verfahren nach Anspruch 1 bis 3, das die Identifizierung von Archaeolen umfasst.

7. Verfahren nach einem der Ansprüche 1 bis 6, worin die Analyse in Schritt (a) auf die Öl-Wasser-Übergangszonen in der Formation konzentriert wird.

8. Verfahren nach Anspruch 1, worin geochemische Hilfsstoffe eingesetzt werden, um zu bestimmen, ob die Formation aktiv abgebaut wird.

9. Verfahren nach einem der Ansprüche 1 bis 8, worin Schritt (e) und Schritt (f) die Charakterisierung der Mikroorganismen unter Anwendung genetischer Charakterisierungsverfahren umfasst.

10. Verfahren nach Anspruch 9, worin die genetischen Charakterisierungsverfahren einen Vergleich der Sequenzen von Genfragmenten, die aus Proben der Mikroorganismen gewonnen wurden, mit Sequenzen bekannter Mikroorganismen umfasst.

11. Verfahren nach einem der Ansprüche 1 bis 10, worin der Schritt des Modifizierens des Umfelds der Formation das Einbringen eines Additivs umfasst, das aus folgenden ausgewählt ist:
(a) Hauptnährstoffen;
(b) Vitaminen;
(c) Spurenelementen;
(d) Puffern;
(e) Wassern
(i) mit unterschiedlichem Salzgehalt;
(ii) mit unterschiedlichem pH-Wert;
(iii) die Komplexbildner enthalten;
(f) Hemmern von Wasserstoff oxidierenden Organismen.

12. Verfahren nach Anspruch 11, worin das Umfeld der Formation durch das Zusetzen von Phosphor modifiziert wird.

13. Verfahren nach Anspruch 11, worin das Umfeld der Formation durch das Zusetzen von Ammoniumionen modifiziert wird.

14. Verfahren nach einem der Ansprüche 1 bis 13, worin der Schritt des Modifizierens des Umfelds der Formation das Verteilen von Gas umfasst.

15. Verfahren nach einem der Ansprüche 1 bis 14, worin die Wasserstoffproduktion durch die Anwendung einer geschichteten Reservoirstruktur gefördert wird, wodurch die Wasserstoffproduktion in einer oberen Reservoirschicht durch die Entfernung von Wasserstoff unter Einsatz eines in einer unteren Reservoirschicht produzierten oder in diese eingeleiteten Gases aufrechterhalten und gefördert wird.

16. Verfahren nach einem der Ansprüche 1 bis 15, worin der Schritt des Modifizierens des Umfelds der Formation das Einleiten von reaktivem, flüssigem organischem Material in die Formation umfasst.

## Revendications

1. Procédé de stimulation de la production d'hydrogène microbien dans une formation souterraine contenant du pétrole, dans lequel un consortium microbien comprenant un ou plusieurs organismes consommant de l'hydrogène est présent, comprenant:
(a) analyser un ou plusieurs composants de la formation pour déterminer les caractéristiques de l'environnement de formation;
(b) détecter la présence d'un consortium microbien, comprenant au moins un micro-organisme fermentatif syntrophique, dans la formation;
(c) établir si les micro-organismes de formation sont actuellement actifs;
(d) déterminer si le consortium microbien comprend un ou plusieurs micro-organismes fermentatifs syntrophiques;
(e) caractérisation d'un ou de plusieurs microorganismes fermentatifs syntrophiques du consortium, et comparer un ou plusieurs organismes **caractérisés** avec au moins un microorganisme **caractérisé** connu ayant une ou plusieurs caractéristiques physiologiques et écologiques connues;
(f) caractérisations desdits un ou plusieurs microorganismes consommant de l'hydrogène, comme des méthanogènes, réducteurs de sulfate ou autres organismes oxydant l'hydrogène du consortium, et comparer un ou plusieurs microorganismes **caractérisés** avec au moins un microorganisme **caractérisé** connu ayant une ou plusieurs caractéristiques physiologiques et écologiques connues;
(g) utiliser des informations obtenues aux étapes (a) à (e) pour déterminer un environnement écologique qui encourage la dégradation microbienne in situ du pétrole et encourage la génération microbienne d'hydrogène par au moins un micro-organisme fermentatif syntrophique du consortium;
(h) utiliser les informations obtenues aux étapes (a) et (f) pour déterminer un environnement écologique qui inhibe une dégradation microbienne in situ de l'hydrogène par ledit au moins un microorganisme oxydant l'hydrogène du consortium;
(i) modifier l'environnement de formation sur la base des déterminations des étapes (g) et (h) pour stimuler la conversion microbienne du pétrole en hydrogène et pour inhiber la conversion microbienne de l'hydrogène en d'autres composés; et
(j) retrait de l'hydrogène produit par les sites de génération de façon à maintenir une conversion microbienne énergétiquement faisable du pétrole en hydrogène.

2. Procédé selon la revendication 1, dans lequel le retrait de l'hydrogène peut être accompli en utilisant des gaz de purge ou de lavage produits ou injectés dans le réservoir.

3. Procédé selon la revendication 1 ou la revendication 2, où l'étape de détection de la présence de bactéries anaérobies dégradant les hydrocarbures fait partie de l'étape (b).

4. Procédé selon l'une quelconque des revendications 1 à 3, qui comprend l'identification de sous-produits d'une activité microbienne indigène.

5. Procédé selon la revendication 4, dans lequel les produits identifiés comprennent des métabolites de dégradation d'hydrocarbures anaérobies.

6. Procédé selon l'une quelconque des revendications 1 à 3, qui comprend une identification d'archaeols.

7. Procédé selon l'une quelconque des revendications 1 à 6, dans lequel l'analyse à l'étape (a) est focalisée sur les zones de transmission huile-eau dans la formation.

8. Procédé selon la revendication 1, dans lequel des substituts géochimiques sont utilisés pour établir si la formation se dégrade activement.

9. Procédé selon l'une quelconque des revendications 1 à 8, dans lequel les étapes (e) et (f) comprennent la caractérisation de microorganismes en utilisant des méthodes de caractérisation génétiques.

10. Procédé selon la revendication 9, dans lequel les méthodes de caractérisation génétiques comprennent la comparaison de séquences de fragments génétiques échantillonnées de microorganismes avec des séquences de microorganismes connus.

11. Procédé selon l'une quelconque des revendications 1 à 10, dans lequel l'étape de modification de l'environnement de formation comprend l'introduction d'un additif sélectionné parmi:
(a) nutriments majeurs;
(b) vitamines;
(c) éléments trace;
(d) tampons;
(e) eaux de:
(i) salinités différentes;
(ii) valeurs pH différentes;
(iii)contenant des agents complexants;
(f) inhibiteurs d'organismes oxydant l'hydrogène.

12. Procédé selon la revendication 11, dans lequel l'environnement de formation est modifié en ajoutant du phosphore.

13. Procédé selon la revendication 11, dans lequel l'environnement de formation est modifié en ajoutant des ions d'ammonium.

14. Procédé selon l'une quelconque des revendications 1 à 13, dans lequel l'étape de modification de l'environnement de formation comprend le lavage au gaz.

15. Procédé selon l'une quelconque des revendications 1 à 14, dans lequel la production de l'hydrogène est encouragée par l'utilisation d'une configuration de réservoir à couches moyennant quoi la génération de l'hydrogène dans une couche de réservoir supérieur est maintenue et encouragée par le retrait de l'hydrogène en utilisant du gaz produit ou injecté dans une couche de réservoir inférieure.

16. Procédé selon l'une quelconque des revendications 1 à 15, dans lequel l'étape de modification de l'environnement de formation comprend l'injection d'une matière organique liquide réactive dans la formation.
